# NEW EUROPEAN PATENT SPECIFICATION

(11) **EP 0 140 410 B2**
(45) Date of publication and mention of the opposition decision: **04.12.1996**
(45) Mention of the grant of the patent: 17.11.1988
(21) Application number: 84201301.3
(22) Date of filing: 10.09.1984
(51) Int. Cl.: C12N 9/30, C12N 9/34, C12P 19/20

(54) **Novel enzyme product and its use in the saccharification of starch**
Enzymprodukt und seine Verwendung in der Verzuckerung von Stärke
Produit enzymatique et son application dans la saccharification de l'amidon

(30) Priority: 11.09.1983 EP 83201303
(43) Date of publication of application: 08.05.1985
(73) Proprietor: GIST-BROCADES N.V., NL-2600 MA Delft (NL)
(72) Inventor: Ducroo, Paul, F-59133 Phalempin (FR); Labout, Johannes Jacobus Maria, NL-2106 EK Heemstede (NL); Noordam, Bertus, NL-2692 BJ 's-Gravenzande (NL)
(74) Representative: Armitage, Ian Michael

(56) References cited:
- EP-A- 0 063 909
- GB-A- 1 037 710
- GB-A- 1 106 421
- GB-A- 2 016 476
- US-A- 4 017 363
- US-A- 4 284 722
- DIE STÄRKE, vol. 27, no. 11, November 1975, pages 377-383, Weinheim, DE; J.J. MARSHALL: "Starch-degrading enzymes, old and new"
- STARCH/STÄRKE, vol. 30, no. 8, 1978, pages 272-275, Verlag Chemie GmbH, Weinheim, DE; N. RAMACHANDRAN et al.: "Studies on the thermophilic amylolytic enzymes of a strain of Aspergillus niger"
- CHEMICAL ABSTRACTS, vol. 73, no. 17, 26th October 1970, page 35, no. 84092z, Columbus, Ohio, USA; J.J. MARSHALL et al.: "Incomplete conversion of glycogen and starch by crystalline amyloglucosidase and its importance in the determination of amylaceous polymers"
- STARCH/STÄRKE, vol. 30, no. 10, 1978, pages 341-345, Verlag Chemie GmbH, Weinheim, DE; P. FINCH et al.: "Comparative studies on glucoamylases isolated from a strain of Aspergillus"
- CHEMICAL ABSTRACTS, vol. 96, no. 3, 18th January 1982, page 208, no. 17141b, Columbus, Ohio, USA; G.I. KVESITADZE et al.: "Selection of microscopic fungi producing acid-stable alpha-amylase and glucoamylase"
- DIE STÄRKE, vol. 27, no. 4, 1975, pages 123-128, Weinheim, DE; S. UEDA et al.: "Multiple forms of glucoamylase of rhizopus species"

## Description

### Field of the invention

The present invention relates to enzymatic starch degradation. More specifically, the invention provides a novel enzyme product useful in the saccharification of starch, especially liquefied starch, and a process for its preparation.

### State of the art

Native starch is known to contain two types of macromolecules composed of glucose units. One type of molecule, called amylose, is linear and consists exclusively of α-1,4-linked glucose units. Starch contains about 25% of amylose. The second type of molecule, called amylopectin, is highly branched and contains α-1,4 as well as α-1,6 linked glucose units. The overall content of α-1,6 linkages is generally less than 5%.

Sugars from starch, in the form of concentrated dextrose syrups, are currently produced at the rate of several million tons per annum by a two stage enzyme catalyzed process involving: (1) liquefaction (or thinning) of solid starch with an α-amylase into dextrins having an average degree of polymerization of about 7-10, and (2) saccharification of the resulting liquefied starch (i.e. starch hydrolysate) with amyloglucosidase, which results in a syrup of high glucose content (92-96% by weight of the total solids). Much of the dextrose syrup produced commercially is then enzymatically isomerized to a dextrose/fructose mixture known as isosyrup.

The two enzymes used, α-amylase and amyloglucosidase, differ in two important aspects. First, α-amylase, which is a so-called endo-enzyme, attacks macro-molecules at random. Amyloglucosidase, on the other hand, is a so-called exo-enzyme and splits glucose units successively from the non-reducing end of the dextrin molecule in the starch hydrolysate. Secondly, α-amylase exclusively attacks α-1,4 linkages whereas amyloglucosidase splits α-1,6 linkages as well.

The recommended name of amyloglucosidase is exo-1,4-α-D-glucosidase, the Enzyme Committee number 3.2.1.3 and the systemic name α-1,4-glucan glucohydrolase. Amyloglucosidase is also called AG or glucoamylase and it will be understood that the terms amyloglucosidase, AG and glucoamylase, as used hereinafter, are synonymous.

Whereas amylopectin is only partially degraded by α-amylase because this enzyme exclusively attacks α-1,4 linkages, substantial hydrolysis of the branched oligosaccharides occurs in the subsequent saccharification step catalyzed by amyloglucosidase which also hydrolyses α-1,6 glucosidic linkages, though at a considerably lower rate than the α-1,4 linkages.

The saccharification stage of the commercial process outlined above has long been recognized to be deficient in certain regards. In particular, the amyloglucosidases currently available catalyse both saccharification and dextrose reversion reactions, e.g. conversion of dextrose into isomaltose, at rates which depend on the substrate concentration. The formation of by-products in this way has limited the saccharification of starch hydrolysates into dextrose to not more than about 95% by weight of dextrose on dry solids basis (hereinafter termed DX) in syrups containing at least 33% dry solids by weight.

It is true that the formation of by-products from reversion reactions may be suppressed by up to about 50% with a concomitant increase of starch conversion of about 1-2% if a relatively high level of amyloglucosidase combined with a dilution of the substrate to about 15% dry solids is employed (cf. US. Patent No. 4,017,363), but the concentration of the resulting dextrose solution to the conventional higher dry solids levels is energy consuming.

In an effect to further increase the DX value it has been proposed to use a debranching enzyme, in conjunction with amyloglucosidase, so as to hydrolyze more efficiently the branched oligosaccharides (containing α-1,6 glucosidic bonds) present in the liquefied starch.

European Patent Application No. 82302001.1, Publication No. 0 063 909, describes a debranching enzyme of the pullulanase type which is produced by a *Bacillus* called *Bacillus acidopullulyticus.* According to this specification the debranching enzyme has optimum activity at a pH in the range of 3.5 to 5.5 (under defined conditions) and its thermal activity optimum at pH 4-5 is at least about 60°C. The residual activity after 72 hours at 60°C at pH 5 is 50% or more. This acid pullulanase is used together with one of the saccharifying enzymes amyloglucosidase or β-amylase. The use of this acid pullulanase in conjunction with amyloglucosidase is reported to result into a higher dextrose level which is higher by about 1% as compared with the level obtained with amyloglucosidase alone under similar conditions. Alternatively the same dextrose level may be achieved using about half the amount of amyloglucosidase.

U.S. Patent No. 4,335,208 discloses the combined action of amyloglucosidase and another debranching enzyme, namely isoamylase from *Pseudomonas amyloderamosa.* According to this reference the isoamylase has a pH optimum close to that of amyloglucosidase so that the amount of the latter can be considerably reduced to obtain the same or even a higher dextrose level than with amyloglucosidase alone. However, the process has a serious drawback in that the isoamylase is heat labile. This means that no saccharification in the presence of isoamylase is technically feasible above about 55°C, whereas amyloglucosidase by itself is normally used at 60°C in the saccharification of starch hydrolysate. Moreover, microorganisms of the genus *Pseudomonas* are not so-called GRAS-microorganisms (Generally Regarded as Safe), so that enzymes produced by such microorganisms are not permitted in food and food processing in the USA.

U.S. Patent No. 3,897,305 discloses the combined use of a amyloglucosidase and pullulanase from *Aerobacrer aerogenes (Klebsiella pneumoniae)* which is stated to give an increase in DX of up to 2% in syrups containing at least 30% dry solids. Practically no saving of amyloglucosidase is achieved however, because of the unfavourable pH optimum (5.5-6.0), of the enzyme from *K. pneumoniae* which makes it necessary to conduct the saccharification at a relatively high pH at which the activity of amyloglucosidase is severely reduced.

Marshall *et al* (Febs Letters, Vol. 9 No. 2, July 1970, pages 85-88) reported that amyloglucosidase obtained from *Aspergillus niger* contained an α-amylase-like impurity apparently essential for complete hydrolysis of starch to glucose. No attempt was however made to characterize or isolate this impurity.

U.S. Patent No. 2,893,921 discloses the production of an amyloglucosidase preparation derived from Aspergillus phoenicis ATCC 13156 and U.S. Patent No. 3,117,063 describes the further refinement of this amyloglucosidase preparation so that it no longer contains transglucosidase activity. Neither of these two U.S. patents report that this amyloglucosidase preparation also contains acid α-amylase.

### Objects of the invention

It is an object of the invention to provide a novel enzyme product having α-glucosidic bond splitting activity at acidic pH, which can be used in the saccharification of starch and, preferably, liquefied starch. It is a further object of the invention to provide a novel process for converting starch into syrups with a high dextrose content.

### The invention

According to its first aspect the present invention provides an enzyme product, other than such product obtained from Aspergillus phoenicis ATCC 13156, comprising amyloglucosidase and acid alpha-amylase, said acid alpha-amylase having substantially alpha-1,4-glucosidic bond splitting activity and showing optimum activity during saccharification reaction at a pH from 3.5 to 5.0 and a temperature from 60 to 75°C, in a ratio of at least 0.16 AAU (acid amylase units) per AGI (amyloglucosidase units), wherein said enzyme product is substantially free from transglucosidase.

This microbial acid amylase is obtainable from amyloglucosidase and has substantial α-1,4-glucosidic bond splitting activity. It effects optimum saccharification at a pH between 3.5 and 5.0 at temperatures from about 60 to about 75°C. Under ordinary storage conditions it is stable over a period of several months.

The acid alpha-amylase occurs as a component in amyloglucosicase preparations and can be obtained in substantially pure form from such preparations using an appropriate separation technique, such as high performance liquid chromatography which is also the preferred method.

Although the novel acid alpha-amylase described below is obtained from a commercially available amyloglucosidase derived from the microorganism *Aspergillus niger,* and tnis is the preferred amyloglucosidase, it will be appreciated that many genera of microorganisms contain species known to produce an amyloglucosidase. Any and all such amyloglucosidases, other than that obtained from Aspergillus phoenicus ATCC 13156, can be used as the source of the said acid alpha-amylase. Preferably, a fungal amyloglucosidase is used as the source.

The thermostability of the acid alpha-amylase derived from *Aspergillus niger* is better than that at the *A. niger* amylogiucosidase. Also the stability and residual activity of said acid alpha-amylase exceeds the same of said amyloglucosidase.

The novel enzyme product according to the invention may be made by adding the acid alpha-amylase to a known amyloglucosidase preparation so as to increase the acid amylase content of the latter.

Preferably, the amyloglucosidase is an *Aspergillus niger* amyloglucosidase and is enriched with an acid aloha-amylase also derived from *Aspergillus niger.*

Preferably, the acid alpha-amylase is added in substantially pure form to the amyloglucosidase.

Alternatively, an amyloglucosidase producing strain or variant or mutant thereof, preferably belonging to the genus *Aspergillus* and more preferably to the species *A*. *niger*, may be found which producer an amyloglucosidase with a relatively high acid amylase content as compared with the amyloglucosidases known in the art, in which case the enzyme product may be obtained by cultivating the same microorganism in a suitable nutrient medium containing carbon and nitrogen sources and inorganic salts The novel enzyme product may also be prepared by selectively improving the fermentation conditions for acid alpha-amylase or partly inactivating the amyloglucosidase in existing preparations.

The novel enzyme product of the invention comprises at least 0.16 AAU of acid alpha-amylase per AGI. One unit of acid alpha-amylase activity (AAU) as used herein is the amount of enzyme which hydrolyses 1.0 mg of soluble starch (100% of dry matter) per minute under standard conditions (pH 4.2; 60°C) into a product which, after reaction with an iodine solution of known strength, gives an optical density at 620 nm equivalent to that of a colour reference as described in the Iodine Starcn Amylase Test described below. One unit of amyloglucosidase activity (AGI) as used herein is defines as the amount of enzyme that releases 1 µmole of dextrose from soluble starch (100% of dry matter) per minute at 60°C under optimum conditions of starch degradation, as described hereinafter. Preferably, the novel enzyme product contains from about 0.2 to about 4.5 AAU of acid amylase per AGI, more preferably from about 0.3 to about 3.0 AAU per AGI and particularly from about 0.7 to about 1.5 AAU per AGI.

It has been surprisingly found that the amyloglucosidase preparations enriched with acid amylase, when used in the saccharification of liquefied starch, result in unexpectedly and significantly higher dextrose levels at shorter saccharification times. The results are comparable with those obtained by the simultaneous action of amyloglucosidase and acid pullulanase, as described in the aforementioned European Patent Application Publ. No. 0 063 909. under similar conditions.

Accordingly, the invention further provides a process for converting starch into dextrose in the form of a syrup, which comprises saccharifying the starch optionally and preferably after a liquefaction step to form a starch hydrolysate, in the presence of the novel enzyme product, as hereinbefore defined. The use of the new enzyme product in the process has the advantage that substantially lower amounts of amyloglucosidase can be used for saccharification of starch hydrolysates resulting in higher yields of glucose per enzyme unit (AGI). The new enzyme product has also the great advantage that higher substrate concentrations can be used in the saccharification of starch and starch hydrolysates. For example, when a starch hydrolysate is saccharified according to the process of this invention, it contains preferably at least 30% by weight of dry solids. The use of higher substrate concentrations substantially reduces evaporation costs.

The saccharification is suitably carried out at a pH in the range of from 2.5 to 6, preferably of from about 3 to about 5 and more preferably of from about 4.0 to about 4.5. The process is suitably effected at temperatures in the range of from 40 to 70°C, preferably of from about 50 to about 65°C, with reaction times in the range of from 15 to 96 hours to obtain maximum yields.

Preferred dosages of amyloglucosidase for the saccharification of starch hydrolysates are normally in the range of from about 8 to about 30 AGI and preferably from about 14 to about 22 AGI per g of dry solids.

It has also been found that the saccharification of starch or a starch hydrolysate can be further improved, when the process is conducted in the presence of the novel enzyme product as defined hereinbefore, which also contains an effective amount of acid pullulanase. A suitable acid pullulanase which can be used for the purpose of this invention is, for example, an acid pullulanase as described in European Patent Application Publ. No. 0 063 909. Preferred dosages of acid pullulanase which can be used in conjunction with the novel enzyme product are in the range of from 0.005 to 5 pullulanase units (PU), the units being as defined in said European Patent Application. The use of the novel enzyme product in conjunction with acid pullulanase in the process has the advantage that unexpectedly and significantly high dextrose levels can be obtained at short saccharification times.

Another suitable method to determine the amount of acid amylase in enzyme preparations is the modified Phadebas Amylase Test described below. One unit of acid amylase activity (AAU') as used herein is defined as the amount of enzyme that gives one unit of absorbance at 620 nm under modified Phadebas amylase test conditions described below. The value of at least 0.16 AAU of acid amylase per AGI under Iodine Starch Amylase Test conditions, as defined hereinbefore, corresponds with the value of at least 0.12 AAU' of acid amylase per AGI under modified Phadebas Amylase Test conditions. A drawback of the latter method is a synergistic effect which occurs when amyloglucosidase is present. Moreover, it is very difficult or even impossible to automatize this method.

It will be understood that, unless otherwise stated, the AAU values which are mentioned in this specification are expressed in units according to the modified Iodine Starch Amylase Test method.

The following test methods and Examples illustrate the invention.

### Iodine starch amylase test

This method is based on the measurement of the optical density of iodine starch complexes in the presence of an amyloglucosidase inhibitor. Acarbose, Bay g 5421, was used as the amyloglucosidase inhibitor, cf. Schmidt *et al,* Naturwissenschaften 64 (1977) 535.

### Reagents

A 2% solution of soluble starch (Lintner, J. T. Baker Co.) in citrate buffer (0.013 M, pH 4.2).

Iodine stock solution containing 22 g iodine and 44 g potassium iodide per litre of distilled water.

Diluted iodine solution: 4 ml of iodine stock solution and 40 g potassium iodide dissolved in distilled water. Distilled water added up to 1 litre.

Colour reference containing 250 g cobaltous chloride 6 aq. and 38.4 g potassium bichromate per litre in 0.01 N HCl.

### Procedure

The starch solution (20 ml) was preheated at 60°C for 20 min. Starting at time 0 exactly 10 ml of the enzyme sample (containing 1.4-1.8 AAU/ml; room temperature) was added to the substrate solution. If amyloglucosidase is believed to be present in the enzyme sample, the amyloglucosidase inhibitor Bay g 5421 is previously added to the enzyme sample in a concentration of 1 g per AGI. After 20 min. of incubation 1 ml of the solution was transferred to 5 ml of the diluted iodine solution. The optical density was immediately measured at 620 nm in a 1 cm cuvet using distilled water as the blank. This procedure of transferring and measuring was repeated at 1 min. intervals until readings were found which were lower than the readings of the colour reference.

The time T needed to reach the absorbance equal to that of the colour reference was established graphically.

The acid amylase activity in units (AAU) present in the incubation solution was calculated from 400/T in which: 400: mg of soluble starch in the incubation solution T: reaction time needed (min.).

### Modified phadebas amylase test

The standard Phadebas amylase test (Marciniak *et al.,* Starch *34* 442 (1982)) modified for conditions of acidic pH and a temperature of 60°C was effected as follows. In a glass vial with screwed cap 1 ml of enzyme sample containing 10 AGI and 4.0 ml acetate buffer (0.3M, pH 4.0) were pipetted. Then a Phadebas tablet (Pharmacia, batch no. HE 74112) was added and after vortexing for 15 sec. the vial was closed and placed in a water bath at 60°C. The reaction was stopped exactly 15 min. after the addition of the tablet by adding 0.3 N NaOH (5 ml) and shaking. After centrifugation the supernatant was removed and the optical density (OD) was measured (in the range 0.2 to 2.0) in a 1 cm cuvet at 620 nm relative to destilled water. A blank (distilled water) underwent the same procedure. The ΔOD is a measure of the acid amylase activity. One unit of acid amylase activity (AAU') is defined as the amount of enzyme that gives one unit of absorbance (ΔOD=1) at 620 nm under these test conditions.

### Amyloglucosidase assay

Soluble starch (2 ml; Lintner Starch, J. T. Baker Co.) in a concentration of 16 g/l of acetate buffer (0.04M, pH 4.3) was preheated at 60°C for 5 min. and then added to 2 ml of enzyme solution (0.15-0.55 AGl/ml). After mixing the suspension was incubated at 60°C. The reaction was terminated after 15 min. by adding 20 ml NaOH (0.005 N) and the glucose concentration determined by the glucose oxidase method.

### The saccharification process

The saccharification process was effected on maltodextrin MDO3 (Roquette Frères) having a dextrose equivalent (DE) of 16.5. This substrate contains some oligosaccharides having fructosyl end groups, from which as much as 0.4-0.5% of the disaccharide maltulose is formed in the saccharification step. To a solution of this substrate (33% dry solids) 2100 AGl/100 g dry solids were added. The pH was adjusted to 4.20 with 1 N acetic acid. The mixture was incubated ato 60°C in a water bath. Aliquots of 0.1 ml were taken from the reaction mixture at 16, 24, 48, 64, 72, 80 and 92 hr and added to 3 ml of distilled water in a closed test tube.

Each diluted sample was immediately placed into a boiling water bath for 10 min. in order to inactivate the enzyme. After cooling about 150 mg of dried Amberlite MB-3 resin (BDH) were added to each sample in order to remove HPLC disturbing salts. After standing for 1 hr the resin was removed and 40 µl of sample were injected onto the HPLC column for glucose determination according to the method of Scobell *et al.* (Cereal Chem., *54* (4), (1977) 905-917), modified in that a Bio-Rad HPX-87C 300 mm column was used. The precision and accuracy of the assay were found to be 0.1% and 0.2% absolute respectively at a glucose concentration in the range of 90-96%.

Under these conditions a peak level of 94.%-94.8% of glucose was achieved using current commercial amyloglucosidase preparations from Miles (Diazyme and Optidex), Novo (AMG) and Gist-Brocades (Amigase GM).

### Example I

### Isolation and identification of acid amylase

In order to identify and isolate amylolytic components present in an amyloglucosidase preparation, an amyloglucosidase enzyme preparation produced by a transglucosidase negative strain of *A*. niger was subjected to high performance liquid chromatography. The system comprised an anion exchange column and a gel filtration column coupled in series. After injection of a part of the AG preparation onto the ion exchange column, the solvent, which was 0.05 M sodium acetate buffer with a pH of 4.0, was led through both columns until the positively charged and uncharged components had reached the gel column. The molecules adsorbed on the ion exchange column were eluted by a salt gradient (0.05-1.65 M sodium acetate buffer, pH 4.0) and then the molecules bound on the gel column were eluted with the original solvent.

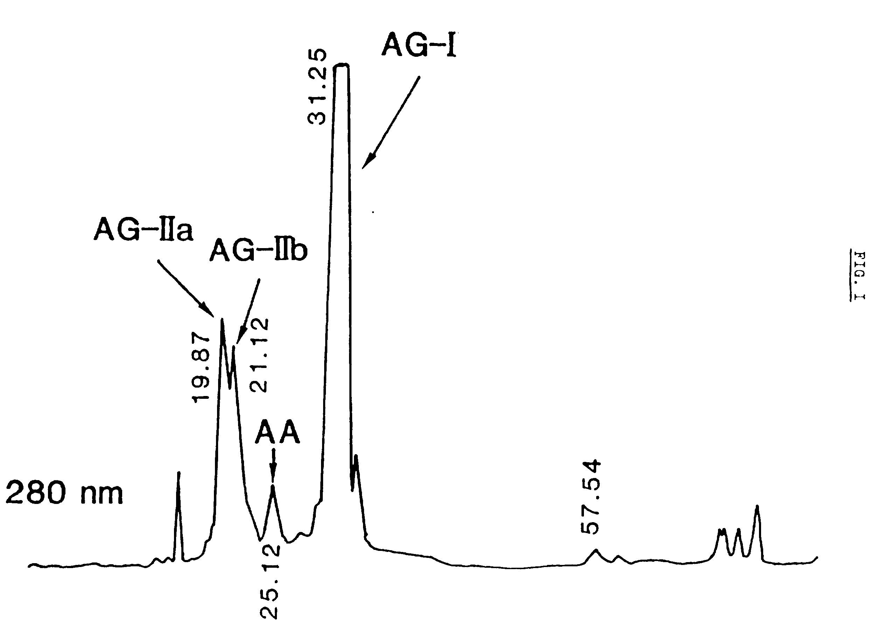

This procedure revealed an excellent separation as can be seen from the accompanying Fig. 1, between the amylolytic enzymes, and both amyloglucosidase isomers and an α-amylase were identified by fractionating the effluent and incubating the collected fraction with suitable substrates, i.e. maltose and soluble starch (10% dry solids). The isolated amyloglucosidase components produced glucose from starch and maltose, whereas the isolated α-amylase produced a typical oligosaccharide pattern from starch.

The characteristics of the α-amylase, in particular in relation to pH and temperature, were determined using soluble starch as a substrate. The amounts of the main products formed (di- and trisaccharides) showed an optimum at a pH of 3.5 to 5.0 indicating that the enzyme is a true acid amylase (AA). The effect of temperature was investigated at a pH of 4.0 and the acid amylase had its optimum between 65 and 70°C as was determined from the behaviour of the trisaccharides formed. These results indicate that the acid amylase is sufficient stable at the standard saccharification temperature of 60°C. The fractions containing AA, the activity of which was stable for more than 3 months, were used for enrichment experiments.

### Example II

### Saccharification with acid amylase enriched samples

Saccharification was accomplished on maltodextrin MDO3 having a dextrose equivalent (DE) of 16.5, and, to a solution of this substrate (33% dry solids), 2100 AGI/100 g dry solids were added. Also, various amounts of acid amylase were added, the activity of which was previously determined according to Phadebas method described above. During the saccharification, the starch hydrolysate was maintained at a pH of 4.0-4.2 anf a temperature of 60°C. Under these conditions the degree of saccharification or glucose formation was measured over the period between 17 and 91 hrs. The experiments with acid amylase enriched samples which are completely free of transglucosidase demonstrated that the glucose yield was increased and the saccharification time was shortened as can be seen from the results of Table I below.

**TABLE I**

| | | | | Glucose yield (%) | | | | | | |
|---|---|---|---|---|---|---|---|---|---|---|
| Sample No. | AAU'/AGI (a) | AAU'/AGI (b) | AAU/AGI (c) | Saccharification time in hr. | | | | | | |
| | | | | 17 | 24 | 41 | 48 | 65 | 72 | 91 |
| 1 | 0.08 | - | 0.074 | 89.1 | 92.5 | 94.1 | 94.1 | 94.7 | 94.7 | 94.5 |
| 2 | 0.08 | 0.07 | 0.20 | 90.7 | 93.6 | 94.6 | 94.8 | 94.7 | 94.7 | 94.5 |
| 3 | 0.08 | 0.15 | 0.35 | 91.7 | 94.2 | 94.9 | 94.8 | 95.0 | 94.8 | 94.6 |
| 4 | 0.08 | 0.35 | 0.73 | 92.7 | 94.6 | 94.9 | 94.9 | 94.6 | 94.8 | 94.5 |
| 5 | 0.08 | 0.70 | 1.40 | 93.5 | 95.0 | 94.8 | 94.8 | 95.0 | 94.8 | 94.2 |
| 6 | 0.08 | 1.50 | 2.92 | 94.0 | 94.9 | 94.8 | - | 94.4 | 94.4 | 94.3 |
| 7 | 0.08 | 2.32 | 4.48 | 94.4 | 95.1 | 94.7 | - | 95.0 | 94.5 | 94.4 |

| | | | | | | | | | | |
|---|---|---|---|---|---|---|---|---|---|---|
| (a): present in original amyloglucosidase preparation (measured with modified Phadebas method) | | | | | | | | | | |
| (b): AAU' of added acid amylase (measured with modified Phadebas method) | | | | | | | | | | |
| (c): acid amylase activities converted into AAU (as defined hereinbefore in the Iodine Starch Amylase Test). | | | | | | | | | | |

The results of Table I show that acid amylase increases the yield of glucose from 94.7% to 95.1% under these conditions while the saccharification time for optimum yields decreased from 70 hours to 24 hours. This makes the use of acid amylase commercially important and equivalent to the results obtained with pullulanase. A portion of the amyloglucosidase can be replaced with the acid amylase while obtaining economically attractive glucose yields and saccharification times.

### Example III

Using the saccharification procedure of Example I, tests were run with the normal and one half normal dosage of amyloglucosidase and with one half normal dosages of amyloglucosidase with enrichment of acid amylase and acid pullulanase, described in European Patent Application Publication No. 0 063 909, alone and in combination. 1 pullulanase unit (PU) is defined as the amount of enzyme necessary to produce 1 µmole of reducing sugar from pullulan per minute under standard conditions. The results are reported in Table II.

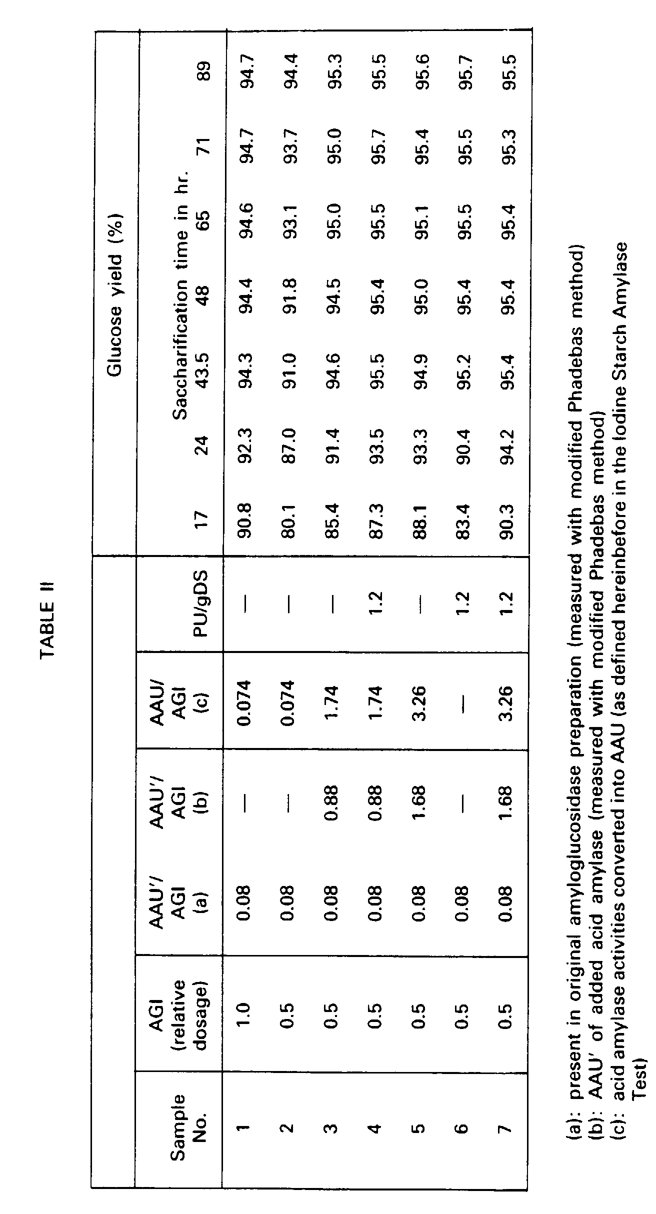

The results of Table II show that the glucose level reached its peak after about 80 hours using half the normal dosage amyloglucosidase (AG) and an acid amylase (AA) enrichment factor, which is slightly longer than the 70 hour saccharification time using the normal amyloglucosidase dosage. However, the peak level of glucose increased from 94.7% to 95.5% which may be due to less isomaltose formation consequent upon the smaller amount of amyloglucosidase used.

Similar results were obtained when pullulanase and amyloglucosidase were used together, although significant differences in glucose production were noted at the shorter saccharification times. The combination of acid amylase, pullulanase and one half of amyloglucosidase showed a faster saccharification resulting in higher yields of glucose per enzyme activity (AGI) per hour.

These results indicate that acid amylase substantially contributes to the hydrolysis of starch in the saccharification step. This surprising effect competes with that of the acidic pullulanse, although the two enzymes act by basically different mechanisms. While pullulanase is thought to be an endo α-1,6 bond splitter, acid amylase has α-1,4 bond splitting activity.

### Example IV

Using the novel acid amylase in the saccharification of starch makes possible an increase in the glucose peak levels, a shortening of the saccharification times and a reduction of the necessary amyloglucosidase/dry solids (DS) ratio. Another advantage in using acid amylase in the saccharification of liquefied starch is the increase in the substrate concentration which is then possible, which can substantially reduce evaporation costs.

Solutions containing substrate (MDO3) in various dry matter contents were adjusted to pH 4.2 and heated to 60°C. Half normal AG dosages (10.5 AGl/gDS) and a 9-fold amount of acid amylase were added. Aliquots were taken at various intervals and analyzed as described in Example I. Control experiments with normal and halved AG dosages without additional acid amylase were also carried out. The results are given in Table III below.

**TABLE III**

| %DS*) | AGl/gDS | AAU/AGI | Saccharification time (h) | Maximum glucose (%) |
|---|---|---|---|---|
| 25 | 10.5 | 0.074 | 140-165 | 95.6 |
| 25 | 10.5 | 0.74 | 64 | 96.1 |
| 29 | 10.5 | 0.074 | 140-165 | 95.3 |
| 29 | 10.5 | 0.74 | 90 | 95.8 |
| 33 | 10.5 | 0.074 | 140-165 | 94.6 |
| 33 | 10.5 | 0.74 | 90 | 95.2 |
| 37 | 10.5 | 0.074 | 140-165 | 93.9 |
| 37 | 10.5 | 0.74 | 71 | 94.7 |
| 45 | 10.5 | 0.074 | 140-165 | 91.3 |
| 45 | 10.5 | 0.74 | 71 | 92.8 |
| 33 | 21 | 0.074 | 71 | 94.7 |

| | | | | |
|---|---|---|---|---|
| *) maltodextrin MDO3 | | | | |

The data in Table II show that when amyloglucosidase is used in conjunction with the new acid amylase the dry matter content (DS) can be elevated to yield maximum glucose levels which are higher than those obtained under similar conditions using commercial amyloglucosidase preparations. For example, a glucose peak level of 94.7% was obtained with a commercial amyloglucosidase preparation at 33% DS. The same maximum glucose level was achieved at the same incubation time with a 10 fold addition of acid amylase and half the amount of AG at 37% DS.

### Example V

Acid α-amylases from other sources, i.e. bacterial enzymes, which are active in the acidic pH range and at 60°C, can also be used to improve the saccharification brought about by amyloglucosidase. Thus a crude fermentation sample of the bacterium ATCC 31199 (see British Specification No. 1539694 CPC International), containing α-amylase activity, was used in a saccharification experiment with amyloglucosidase. Using the crude sample in a ratio of AAU/AGI=0.74 gave significantly higher glucose levels compared with those obtained with amyloglucosidase only in a control experiment, although the values were lower than those obtained by a corresponding amount of the fungal acid amylase. The results are shown in the following Table IV.

**TABLE IV**

| | | | Glucose yield (%) | | | | | | | |
|---|---|---|---|---|---|---|---|---|---|---|
| Sample | AAU/AGI | AGl/gDS | Saccharification time in hr. | | | | | | | |
| | | | 24 | 40 | 47 | 64 | 71 | 88 | 95 | 112 |
| AG | 0.074 | 10.5 | 80.2 | 88.9 | 90.3 | 92.2 | 92.8 | 93.6 | 94.0 | 94.3 |
| AG+Bacterial AA | 0.74 | 10.5 | 81.7 | 90.4 | 91.6 | 93.1 | 93.5 | 94.1 | 94.2 | 94.6 |
| AG+Fungal AA (cf. Example I) | 0.74 | 10.5 | - | 94.4 | 94.7 | 95.1 | 95.2 | 95.2 | - | - |

### Example VI

Saccharification experiments were performed by the same procedure as described hereinbefore. Solutions containing substrate (MDO3, 33% DS) were adjusted to pH values between 3.5 and 5.0 and heated to 60°C. Amyloglucosidase (21 AGI/g DS) together with a 9-fold amount of AA (compared with the amount present in the AG preparation) were added. Aliquots were taken at various intervals and analyzed. Control experiments were also performed. The following data were obtained, see Table V.

**TABLE V**

| Starting pH | pH after 94 h | Saccharification time (h) | Glucose peak level (%) |
|---|---|---|---|
| 3.5 | 3.45 | 71 | 95.3 |
| 4.0 | 3.85 | 64 | 95.3 (94.9)* |
| 4.2 | 3.95 | 64 | 95.3 (94.8)* |
| 4.5 | 4.1 | 64 | 95.3 (94.6)* |
| 5.0 | 4.2 | 64 | 95.2 |

| | | | |
|---|---|---|---|
| *Controls (AG dosages without extra AA addition) | | | |

Thus, using excess of acid amylase comparable glucose peak levels were obtained in the pH range of 3.5 to 5.0.

### Example VII

Solutions containing substrate (MDO3, 33% DS) were adjusted to pH 4.2 and heated to various temperatures. Amyloglucosidase (21 AGI/gDS) and a 9-fold amount of acid amylase were added. Aliquots were taken and analyzed as described in Example II. Controls (AG dosages without extra AA addition) were also carried out. The results are given in Table VI below.

**TABLE VI**

| Temperature (°C) | AAU/AGI | Saccharification time in hr. | Glucose yield (%) |
|---|---|---|---|
| 55 | 0.074 | 65 | 94.6 |
| | 0.74 | 47 | 95.0 |
| 57.5 | 0.074 | 71 | 95.0 |
| | 0.74 | 47 | 95.5 |
| 60 | 0.074 | 71 | 94.8 |
| | 0.74 | 64 | 95.3 |
| 62.5 | 0.074 | 90 | 94.8 |
| | 0.74 | 64 | 95.4 |
| 65 | 0.074 | 117 | 93.0 |
| | 0.74 | 89 | 94.6 |

These results confirm that acid amylase is stable at temperatures up to at least 65°C, which makes it very suitable for use in conjunction with amyloglucosidase at relatively high saccharification temperatures. The lower glucose values at 65°C are likely caused by the lower thermostability of the AG enzyme relative to AA. The presence of acid amylase has a beneficial effect on the glucose production at higher temperature.

## Claims

1. An enzyme product, other than such product obtained from Aspergillus phoenicis ATCC 13156, comprising amyloglucosidase and acid alpha-amylase, said acid alpha-amylase having substantially alpha-1,4-glucosidic bond splitting activity and showing optimum activity during saccharification reaction at a pH from 3.5 to 5.0 and a temperature from 60 to 75°C, in a ratio of at least 0.16 AAU (acid amylase units) per AGI (amyloglucosidase units), wherein said enzyme product is substantially free from transglucosidase.

2. The enzyme product according to claim 1 containing 0.2 to 4.5 AAU per AGI.

3. The enzyme product according to claim 1 or 2 wherein the amyloglucosidase is derived from Aspergillus niger.

4. The enzyme product according to any one of claims 1 to 3 wherein the acid alpha-amylase is derived from Aspergillus niger.

5. The enzyme product according to any one of claims 1 to 4 also containing an effective amount of acid pullulanase.

6. The enzyme product according to claim 5 wherein the acid pullulanase is producible by Bacillus acidopullulyticus.

7. A process for converting starch into dextrose in the form of a syrup which comprises saccharifying starch or a starch hydrolysate in the presence of an enzyme product as defined in any one of claims 1 to 6.

8. The process according to claim 7 wherein a starch hydrolysate containing at least 30% by weight of dry solids is saccharified.

9. The process according to claim 7 or 8 wherein the saccharification is conducted in the pH-range of from 3 to 5 and at a temperature in the range of from 40 to 70°C.

10. The process according to claim 9 wherein the saccharification is conducted at pH 4 to 4.5 and a temperature of 50 to 65°C.

11. The process according to any of claims 7 to 10 wherein the amount of amyloglucosidase used is from 8 to 30 AGI per g of total dry solids.

12. The process according to claim 11 wherein the amount of amyloglucosidase used is from 14 to 22 AGI per g of total dry solids.

13. The process according to any one of claims 7 to 12 wherein the saccharification is conducted in the presence of acid pullulanase.

## Patentansprüche

1. Enzymprodukt, das Amyloglucosidase und saure α-Amylase in einem Verhältnis von mindestens 0,16 AAU (saure Amylaseeinheiten) pro AGI (Amyloglucosidaseeinheiten) aufweist, wobei die saure α-Amylase im wesentlichen α-1,4-glucosidische Bindungen spaltende Aktivität hat und eine optimale Aktivität während der Verzuckerungsreaktion bei einem pH von 3,5 bis 5,0 und einer Temperatur von 60 bis 75°C zeigt, und wobei das besagte Enzymprodukt im wesentlichen frei von Transglucosidase ist.

2. Enzymprodukt nach Anspruch 1, das 0,2 bis 4,5 AAU pro AGI enthält.

3. Enzymprodukt nach Anspruch 1 oder 2, worin die Amyloglucosidase von Aspergillus niger stammt.

4. Enzymprodukt nach einem der Ansprüche 1 bis 3, worin die saure α-Amylase von Aspergillus niger stammt.

5. Enzymprodukt nach einem der Ansprüche 1 bis 4, das auch eine wirksame Menge saure Pullulanase enthält.

6. Enzymprodukt nach Anspruch 5, worin die saure Pullulanase durch Bacillus acidopullulyticus erzeugbar ist.

7. Verfahren zur Überführung von Stärke in Dextrose in Form eines Sirups, dadurch gekennzeichnet, dass man Stärke oder ein Stärkehydrolysat in Gegenwart eines Enzymproduktes nach einem der Ansprüche 1 bis 6 verzuckert.

8. Verfahren nach Anspruch 7, worin ein Stärkehydrolysat verzuckert wird, das mindestens 30 Gew.-% Trockensubstanz enthält.

9. Verfahren nach Anspruch 7 oder 8, worin die Verzuckerung im pH-Bereich von 3 bis 5 und bei einer Temperatur im Bereich von 40 bis 70°C ausgeführt wird.

10. Verfahren nach Anspruch 9, worin die Verzuckerung bei pH 4 bis 4;5 und einer Temperatur von 50 bis 65°C ausgeführt wird.

11. Verfahren nach einem der Ansprüche 7 bis 10, worin die verwendete Menge Amyloglucosidase 8 bis 30 AGI pro g Gesamttrockensubstanz beträgt.

12. Verfahren nach Anspruch 11, worin die verwendete Menge Amylogucosidase 14 bis 22 AGI pro g Gesamttrockensubstanz beträgt.

13. Verfahren nach einem der Ansprüche 7 bis 12, worin die Verzuckerung in Gegenwart von saurer Pullulanase ausgeführt wird.

## Revendications

1. Produit enzymatique comprenant de l'amyloglucosidase et de l'alpha-amylase acide, laquelle alpha-amylase acide a une activité sensible de coupure de la liaison alpha-1,4-glucosidique et manifeste une activité optimale pendant la réaction de saccharification à un pH de 3,5 à 5,0 et une température de 60 à 75°C, dans un rapport d'au moins 0,16 UAA (unités d'amylase acide) par AGI (unités d'amyloglucosidase), où le produit enzymatique est sensiblement exempt de transglucosidase.

2. Produit enzymatique suivant la revendication 1, contenant 0,2 à 4,5 UAA par AGI.

3. Produit enzymatique suivant la revendication 1 ou 2, dans lequel l'amyloglucosidase provient d'Aspergillus niger.

4. Produit enzymatique suivant l'une quelconque des revendications 1 à 3, dans lequel l'alpha-amylase acide provient d'Aspergillus niger.

5. Produit enzymatique suivant l'une quelconque des revendications 1 à 4, contenant aussi une quantité efficace de pullulanase acide.

6. Produit enzymatique suivant la revendication 5, dans lequel la pullulanase acide est susceptible d'être produite par Bacillus acidopullulyticus.

7. Procédé pour convertir l'amidon en dextrose sous la forme d'un sirop, qui comprend la saccharification de l'amidon ou d'un hydrolysat d'amidon en présence d'un produit enzymatique suivant l'une quelconque des revendications 1 à 6.

8. Procédé suivant la revendication 7, dans lequel un hydrolysat d'amidon contenant au moins 30% en poids de solides secs est saccharifié.

9. Procédé suivant la revendication 7 ou 8, dans lequel la saccharification est effectuée dans l'intervalle de pH de 3 à 5 et à une température dans l'intervalle de 40 à 70 °C.

10. Procédé suivant la revendication 9, dans lequel la saccharification est effectuée à un pH de 4 à 4,5 et à une température de 50 à 65°C.

11. Procédé suivant l'une quelconque des revendications 7 à 10, dans lequel la quantité d'amyloglucosidase utilisée se situe entre 8 et 30 AGI par gramme de solides secs totaux.

12. Procédé suivant la revendication 11, dans lequel la quantité d'amyloglucosidase utilisée est de 14 à 22 AGI par gramme de solides secs totaux.

13. Procédé suivant l'une quelconque des revendications 7 à 12, dans lequel la saccharification est effectuée en présence de pullulanase acide.
